# EUROPEAN PATENT APPLICATION

(11) **EP 2 327 783 A1**
(43) Date of publication of application: **01.06.2011**
(21) Application number: 09177297.0
(22) Date of filing: 27.11.2009
(51) Int. Cl.: C12N 15/113, A61K 31/713, A61K 31/711, A61P 9/00

(54) **Pharmaceutical composition comprising miRNA-100 and its use in the modulation of blood vessel growth**

(71) Applicant: Universitätsklinikum Freiburg, 79106 Freiburg (DE)
(72) Inventor: Grundmann, Sebastian, 79102 Freiburg (DE); Moser, Martin, 79106 Freiburg (DE); Bode, Christoph, 79108 Freiburg (DE)
(74) Representative: Keller, Günter

(57) **Abstract**

Disclosed is a pharmaceutical composition comprising a miRNA-100 molecule or an antagomir thereof or a variant thereof.

## Description

Micro RNAs (miRNAs) are a class of small non-protein encoding RNAs that can inhibit translation. These RNA species that are expressed from specialized genes have important roles in the regulation of cellular gene expression, including the regulation of development, the differentiation of hematopoetic stem cells, apoptosis and the development of cancer. miRNAs are transcribed individually or in combination into long primary transcripts (pri-miRNAs) by RNA polymerase ll or lll. It is assumed that the human genome contains at least 500 distinct miRNA genes which are initially expressed as 5'-capped and polyadenylated RNA polymerase ll transcripts. They are expressed either as individually regulated genes or as clusters of miRNAs and then processed from single primary transcript that may contain several miRNAs. "Drosha" cleaves pri-miRNAa into 70-100 nucleotide pre-miRNAs that form specific secondary hairpin-loop structures. Then cytoplasmatic processing occurs to the about 16 to 29 nucleotide single-stranded mature form by the enzyme called "dicer". The so formed double stranded RNA molecules which contain the mature miRNA and its antisense strand are then unwound by a helicase and the mature miRNA is loaded onto the RNA induced silencing complex (RISC) while the antisense strand is degraded.

The translational silencing is effected either by inhibiting protein synthesis after binding via incomplete base pairing to the 3' untranslated regions (3'-UTR) of target mRNAs and/or by binding to mRNAs with perfect complementary, which leads to cleavage of the targeted mRNA. Therefore miRNA mediated inhibition of gene expression can potentially regulate many cellular targets, and individual genes can be targeted by multiple miRNAs.

### Summary of the invention:

The adaptive growth of blood vessels is an important protective mechanism in cardiovascular disease; the underlying regulatory mechanisms of this process however are only partly understood. Recently, small endogenous RNAs were found to play an important role in embryonic and postnatal blood vessel development. MicroRNA-transcriptome analysis was used to screen for miRNAs differentially expressed following induction of hindlimb ischemia in mice, to identify miRNAs involved in angiogenic and arteriogenic regulation. It was found that the downregulated microRNA-100 modulates proliferation, tube formation and sprouting activity of endothelial cells and functions as an endogenous repressor of the serine/threonine protein kinase mTOR. Overexpression of miR-100 attenuated cellular proliferation and this defect could be rescued by simultaneous transfection with an mTOR-construct lacking the miRNA-binding site. MiR-100 inhibition by specific antagomirs in vivo resulted in a stimulation of angiogenesis following femoral artery occlusion in mice, whereas treatment with the mTOR-inhibitor rapamycin had the opposite effect. Downregulation of miR-100 in endothelial cells was induced by increased levels of the pro-arteriogenic cytokine TNF-alpha, which expression was inversely correlated with miR-100 levels in ischemic tissue.

An anti-angiogenic function of miR-100 at least partly via repression of mTOR-signalling was demonstrated. Inhibition or enhancement of miR-100 is therefore a novel approach for positive or negative modulation of blood vessel growth and other mTOR-dependent processes.

The adaptive growth of blood vessels is an important protective mechanism in patients with chronic vascular occlusive disease. The progressive occlusion of a major artery results in hemodynamic changes and downstream tissue ischemia that induce both the proliferation of small pre-existing collateral arteries (arteriogenesis) as well as capillary sprouting in ischemic tissue (angiogenesis). In the recent past, transcriptional profiling has been used to investigate the regulatory principles that facilitate both shear-stress dependent arteriogenesis and hypoxia-induced angiogenesis and several key regulators have been identified.

First studies suggest an important regulatory function of miRNA in embryonic and postnatal blood vessel development. The multi-domain protein Dicer is responsible for the processing of miRNA precursor molecules to mature miRNAs and mice with a homozygous mutation of Dicer die early during embryonic development due to vascular defects, suggesting a critical role of miRNAs in embryonic vasculogenesis. Dews et al. Nat. Genet. (2006), 38, 1060-1065 demonstrate that the transduction of carcinoma cells with miR-17-92 results in an increased tumor perfusion and that modulation of miRNAs can be used to enhance (in this case pathological) tissue perfusion.

Several groups worldwide are currently studying miRNA regulation of tumor angiogenesis, but the miRNA expression profile during non-neoplastic blood vessel growth as a compensatory mechanism in vascular occlusive diseases has been little investigated so far. The changes in microRNA-expression during the adaptive neovascularization following vascular occlusion and the functional involvement of microRNAs in modulating angiogenesis and arteriogenesis in a mouse model of peripheral artery occlusive disease are disclosed.

Mourelatos et al. Genes Dev. (2002), 16, 720-728 described miR-100 as a small endogenous RNA and subsequently it was found to be differentially regulated in several studies on tumour expression patterns.

miRNA-100 is localized on chromosome 11 in a cluster with let7a-2 and forms a miRNA-familiy with the sequence related miR-99. In the cardiovascular field, miR-100 was first mentioned in a report by Sucharov et al. J. Mol. Cell Cardiol (2008), 45, 185-192, showing a significant upregulation of miR-100 in tissue samples from failing hearts of patients with idiopathic dilated cardiomyopathy. Besides these differential expression results, the regulation and function of miR-100 was little explored. Recently, Henson et al., Genes Chromosomes Cancer (2009), 48, 569-582 described a decreased expression of miR-100 in oral squamous cell carcinoma and for the first time performed functional studies of this microRNA in cultured cancer cells. They reported a significantly reduced cell proliferation following pre-miR-100 transfection, a finding in good correspondence with the results presented herein.

The potential functional importance of miR-100 in cancer biology was further strengthened by a recent report by Shi et al. Int. J. Cancer (2009) (Int J Cancer. 2009 Sep 8. [Epub ahead of print], PMID: 19739117), showing that decreased expression levels of miR-100 in human nasopharynx carcinoma cells result in disease progression. This was shown to correlate with higher levels of the mitotic regulator polo-like kinase 1; the effects on other potential target genes such as mTOR however were not investigated.

### Preferred embodiments of the invention:

The function of miR-100 in the vascular system has not been investigated so far. Here, the first study applying differential expression results from an in vivo study to identify a new microRNA-regulator of angiogenesis is disclosed. Here, the downregulation of the microRNA-100 following induction of ischemia in vivo and the function of this microRNA as an anti-angiogenic repressor is described.

While miR-100 was not the microRNA with the strongest deregulation following induction of hindlimb ischemia and its expression is not vascular specific, research was focused on this gene because of its persistent decrease in expression during the angiogenic time window and its promising selection of predicted target genes. miR-100 is expressed in both endothelial and vascular smooth muscle cells in vivo and in culture. Both loss-of-function as well as gain-of-function approaches were used to verify a significant regulatory function of this miRNA in several in vitro angiogenesis models as well as on more general cellular functions, such as proliferation. Interestingly, the magnitude of effects of miR-100 inhibition on proliferation in the different cell types correlated with the baseline levels of this microRNA in these cells.

Using a combination of genome-wide expression analysis in endothelial cells following miR-100 overexpression and bioinformatics prediction of miRNA-binding sites, the mammalian target of rapamycin was identified as a direct mRNA target of miR-100. mTOR was previously shown to be required for angiogenesis and endothelial cell proliferation in response to hypoxia and is one of the promising targets for novel anti-angiogenic cancer therapies.

It is demonstrated that miR-100 is able to repress mTOR-dependent cellular proliferation and that this effect can be neutralized by expressing mTOR lacking the miR-100 binding site. While other miR-100 target genes cannot be excluded as mediators of the observed effects on neovascularization, it shows that the repression of mTOR is an important mediatory step of miR-100 function in vascular cells. In addition, histological analysis showed an increased mTOR expression in growing collateral arteries and the treatment with the mTOR inhibitor rapamycin resulted in the opposite effect on hindlimb perfusion restoration as compared to miR-100-specific antagomir treatment.

The present invention adds a new candidate to the list of described "angiomiRs" and illustrates the role of miR-100 in the regulation of cellular proliferation and angiogenesis and its function as an endogenous mTOR-modulator. This implicates an important role of this miRNA also in other physiological and pathophysiological processes. miR-100 is a new promising target for pharmacological therapy in cardiovascular disease.

The present invention provides a pharmaceutical composition which comprises a miRNA-molecule or a sequence complementary thereto or a variant thereof and in particular one or more antagomir. Antagomirs are chemically engineered oligonucleotides which are used to silence endogenous microRNA. An antagomir is a small synthetic RNA that is perfectly complementary to the specific miRNA target with either mispairing at the cleavage site of Ago2 or some sort of base modification to inhibit Ago2 cleavage. Usually, antagomirs have some sort of modification to make it more resistant to degradation. It is unclear how antagomirization (the process by which an antagomir inhibits miRNA activity) operates, but it is believed to inhibit by irreversibly binding the miRNA. Antagomirs are used to constitutively inhibit the activity of specific miRNAs.

The active molecule is based on the sequence of miRNA-100 having the following sequence:
GUGUUCAAGCCUAGAUGCCCAA (SEQ ID No. 1).

The RNA sequence can also be present as DNA sequence whereby the uracil (U) is replaced by tymidin (T). The sequence reads then as follows:
GTGTTCAAGCCTAGATGCCCAA (SEQ ID No. 2).

For the pharmaceutical treatment the following antagomir sequence was preferably used: Antag-100: 5'-cacaaguucggaucuacggguu-3' (SEQ ID No. 3);

As control the sequences SEQ ID NO:4 and NO:5 were used:
Antag-cont1: 5'-aaggcaagcugacccugaaguu-3' (SEQ ID No. 4) and
Antag-cont2: 5'-caccaguuaggcucuacggauu-3' (SEQ ID No. 5).

The present invention relates also to variants of the above mentioned sequences SEQ ID N0:1, SEQ ID NO:2 and SEQ ID NO:3 whereby variant means that one or two nucleotides may be deleted or added or replaced by other nucleotides.

The preferred variants of SEQ ID N0:1 and/or SEQ ID NO:2 are at least 85%, preferably 90% and more preferably 95% homologue to SEQ ID N0:1 and/or SEQ ID NO:2.

The preferred variants of the antagomir sequence of SEQ ID NO:3 are at least 85%, preferred 90% and more preferred 95% homologue to the SEQ ID NO:3. This means that in the variants up to three nucleotides can be replaced by other nucleotides, preferably only two and more preferred only one nucleotide is replaced. The term homology is understood as identity. This means that e.g. at least 85% of the nucleotides are identical whereas the remainder of the nucleotides may be changed.

In a preferred embodiment of the variants, the partial sequence:
'"UAOGGGU (SEQ ID No. 6) is not changed. Therefore, changes in the sequence ID NO:3 occur only in positions of sequence ID NO:3 whch do not correspond with SEQ ID NO:6.

This is also true for the complementary sequence or the corresponding desoxyribonucleotide sequence. In preferred embodiments the bases at position 5, 9, 12 and 20 are not mutated, which means that the bases at the mentioned positions in SEQ ID No. 1 or SEQ ID NO:3 or a sequence complementary thereto remain unchanged.

The biologically active structures as described herein can be introduced into the body of the person to be treated as a nucleic acid wthin a vector which replicates into the host cells and produces the oligonucleotides.

Variants may also comprise the above mentioned sequences and a modified oligonucleotide conjugated to one or more moieties which enhance the activity, cellular distribution or cellular uptake of the resulting antisense oligonucleotides. A preferred moiety is a cholesterol moiety or a lipid moiety. Additional moieties for conjugation include carbohydrates, phospholipids, biotin, phenazine, folate, phenanthridine, anthraquinone, acridine, fluoresceins, rhodamines, coumarins, and dyes. In certain variants, a conjugate group is attached directly to a modified oligonucleotide. In certain variants, a conjugate group is attached to a modified oligonucleotide by a linking moiety selected from amino, hydroxyl, carboxylic acid, thiol, unsaturations (e.g., double or triple bonds), 8-amino-3,6-dioxaoctanoic acid (ADO), succinimidyl 4-(N-maleimidomethyl) cyclohexane-1-carboxylate (SMCC), 6-aminohexanoic acid (ALEX or AHA), substituted C1-C10 alkyl, substituted or unsubstituted C2-C10 alkenyl, and substituted or unsubstituted C2-C10 alkynyl. In certain variants, a substituent group is selected from hydroxyl, amino, alkoxy, carboxy, benzyl, phenyl, nitro, thiol, thioalkoxy, halogen, alkyl, aryl, alkenyl and alkynyl.

In certain variants, the compound comprises a modified oligonucleotide having one or more stabilizing groups that are attached to one or both termini of a modified oligonucleotide to enhance properties such as, for example, nuclease stability. Included in stabilizing groups are cap structures. These terminal modifications protect a modified oligonucleotide from exonuclease degradation, and can help in delivery and/or localization within a cell. The cap can be present at the 5'-terminus (5'-cap), or at the 3'-terminus (3'-cap), or can be present on both termini. Cap structures include, for example, inverted deoxy abasic caps.

Suitable cap structures include a 4',5'-methylene nucleotide, a 1-(beta-D-erythrofuranosyl) nucleotide, a 4'-thio nucleotide, a carbocyclic nucleotide, a 1,5-anhydrohexitol nucleotide, an L-nucleotide, an alpha-nucleotide, a modified base nucleotide, a phosphorodithioate linkage, a threo-pentofuranosyl nucleotide, an acyclic 3',4'-seco nucleotide, an acyclic 3,4-dihydroxybutyl nucleotide, an acyclic 3,5-dihydroxypentyl nucleotide, a 3'-3'-inverted nucleotide moiety, a 3'-3'-inverted abasic moiety, a 3'-2'-inverted nucleotide moiety, a 3'-2'-inverted abasic moiety, a 1,4-butanediol phosphate, a 3'-phosphoramidate, a hexylphosphate, an aminohexyl phosphate, a 3'-phosphate, a 3'-phosphorothioate, a phosphorodithioate, a bridging methylphosphonate moiety, and a non-bridging methylphosphonate moiety 5'-amino-alkyl phosphate, a 1,3-diamino-2-propyl phosphate, 3-aminopropyl phosphate, a 6-aminohexyl phosphate, a 1,2-aminododecyl phosphate, a hydroxypropyl phosphate, a 5'-5'-inverted nucleotide moiety, a 5'-5'-inverted abasic moiety, a 5'-phosphoramidate, a 5'-phosphorothioate, a 5'-amino, a bridging and/or non-bridging 5'-phosphoramidate, a phosphorothioate, and a 5'-mercapto moiety.

The present invention relates to the miRNA-100 molecule, an antagomir thereof or variants as described above, which are used in pharmaceutical compositions and for the preparation of suitable medicaments.

The miRNA-molecule or the variants as described above can be used for the preparation of pharmaceutical compositions which are used for the positive or negative modulation of blood vessel growth.

In a preferred embodiment, the miRNA-100 molecule or the variants thereof are used for the modulation of proliferation, tube-formation and sprouting activity of endothelial cells. The effect thereof is the formation of new blood vessels in particular for the treatment of cardiovascular diseases.

A compound comprising a modified oligonucleotide complementary to a miRNA, or precursor thereof, described herein is prepared as a pharmaceutical composition for the modulation of endothelial cells. Suitable administration routes include, but are not limited to, oral, rectal, transmucosal, intestinal, enteral, topical, suppository, through inhalation, intrathecal, intraventricular, intraperitoneal, intranasal, intraocular and parenteral (e.g., intravenous, intramuscular, intramedullary, and subcutaneous). An additional suitable administration route includes chemoembolization. In certain embodiments, pharmaceutical intrathecals are administered to achieve local rather than systemic exposures. For example, pharmaceutical compositions may be injected directly in the area of desired effect.

In certain embodiments, a pharmaceutical composition of the present invention is administered in the form of a dosage unit (e.g., tablet, capsule, bolus, etc.). In certain embodiments, such pharmaceutical compositions comprise a modified oligonucleotide in a dose selected from 25 mg up to 800 mg. In preferred embodiments, a pharmaceutical composition of the present invention comprises a dose of modified oligonucleotide selected from 25 mg, 50 mg, 75 mg, 100 mg, 150 mg, 200 mg, 250 mg, 300 mg, 350 mg, 400 mg, 500 mg, 600 mg, 700 mg, and 800mg.

A pharmaceutical agent may be a sterile lyophilized modified oligonucleotide that is reconstituted with a suitable diluent, e.g., sterile water for injection or sterile saline for injection. The reconstituted product is administered as a subcutaneous injection or as an intravenous infusion after dilution into saline. The lyophilized drug product consists of a modified oligonucleotide which has been prepared in water for injection, or in saline for injection, adjusted to pH 7.0-9.0 with acid or base during preparation, and then lyophilized. The lyophilized modified oligonucleotide may be 25-800 mg of a modified oligonucleotide. The compositions of the present invention may additionally contain other adjunct components conventionally found in pharmaceutical compositions, at their art-established usage levels. Thus, for example, the compositions may contain additional, compatible, pharmaceutically-active materials such as, for example, antipruritics, astringents, local anesthetics or anti-inflammatory agents, or may contain additional materials useful in physically formulating various dosage forms of the compositions of the present invention, such as dyes, flavoring agents, preservatives, antioxidants, opacifiers, thickening agents and stabilizers. However, such materials, when added, should not unduly interfere with the biological activities of the components of the compositions of the present invention. The formulations can be sterilized and, if desired, mixed with auxiliary agents, e.g., lubricants, preservatives, stabilizers, wetting agents, emulsifiers, salts for influencing osmotic pressure, buffers, colorings, flavorings and/or aromatic substances and the like which do not deleteriously interact with the oligonucleotide(s) of the formulation.

The pharmaceutical compositions of the present invention may comprise one or more modified oligonucleotides and one or more excipients. In certain such embodiments, excipients are selected from water, salt solutions, alcohol, polyethylene glycols, gelatin, lactose, amylase, magnesium stearate, talc, silicic acid, viscous paraffin, hydroxymethylcellulose and polyvinylpyrrolidone.

The pharmaceutical composition of the present invention is prepared using known techniques, including, but not limited to mixing, dissolving, granulating, dragee-making, levigating, emulsifying, encapsulating, entrapping or tabletting processes.

The pharmaceutical composition of the present invention may be a liquid (e.g., a suspension, elixir and/or solution). In certain of such embodiments, a liquid pharmaceutical composition is prepared using ingredients known in the art, including, but not limited to, water, glycols, oils, alcohols, flavoring agents, preservatives, and coloring agents.

The pharmaceutical composition of the present invention may also be a solid (e.g., a powder, tablet, and/or capsule). In certain of such embodiments, a solid pharmaceutical composition comprising one or more oligonucleotides is prepared using ingredients known in the art, including, but not limited to, starches, sugars, diluents, granulating agents, lubricants, binders, and disintegrating agents.

In certain embodiments, a pharmaceutical composition of the present invention is formulated as a depot preparation. Certain such depot preparations are typically longer acting than non-depot preparations. In certain embodiments, such preparations are administered by implantation (for example subcutaneously or intramuscularly) or by intramuscular injection. In preferred embodiments, depot preparations are prepared using suitable polymeric or hydrophobic materials (for example an emulsion in an acceptable oil) or ion exchange resins, or as sparingly soluble derivatives, for example, as a sparingly soluble salt.

In certain embodiments, a pharmaceutical composition of the present invention comprises a delivery system. Examples of delivery systems include, but are not limited to, liposomes and emulsions. Certain delivery systems are useful for preparing certain pharmaceutical compositions including those comprising hydrophobic compounds. In certain embodiments, certain organic solvents such as dimethylsulfoxide are used.

In preferred embodiments, a pharmaceutical composition of the present invention comprises one or more tissue-specific delivery molecules designed to deliver the one or more pharmaceutical agents of the present invention to specific tissues or cell types. For example, in certain embodiments, pharmaceutical compositions include liposomes coated with a tissue-specific antibody.

In certain embodiments, a pharmaceutical composition of the present invention comprises a co- solvent system. Certain of such co-solvent systems comprise, for example, benzyl alcohol, a nonpolar surfactant, a water-miscible organic polymer, and an aqueous phase. In certain embodiments, such co- solvent systems are used for hydrophobic compounds. A non-limiting example of such a co-solvent system is the VPD co-solvent system, which is a solution of absolute ethanol comprising 3% w/v benzyl alcohol, 8% w/v of the nonpolar surfactant Polysorbate 80™ and 65% w/v polyethylene glycol 300. The proportions of such co-solvent systems may be varied considerably without significantly altering their solubility and toxicity characteristics. Furthermore, the identity of co-solvent components may be varied: for example, other surfactants may be used instead of Polysorbate 80™^{;} the fraction size of polyethylene glycol may be varied; other biocompatible polymers may replace polyethylene glycol, e.g., polyvinyl pyrrolidone; and other sugars or polysaccharides may substitute for dextrose.

In preferred embodiments, a pharmaceutical composition of the present invention comprises a sustained-release system. A non-limiting example of such a sustained-release system is a semi-permeable matrix of solid hydrophobic polymers. In certain embodiments, sustained-release systems may, depending on their chemical nature, release pharmaceutical agents over a period of hours, days, weeks or months.

In certain embodiments, a pharmaceutical composition of the present invention is prepared for oral administration. In certain of such embodiments, a pharmaceutical composition is formulated by combining one or more compounds comprising a modified oligonucleotide with one or more pharmaceutically acceptable carriers. Certain of such carriers enable pharmaceutical compositions to be formulated as tablets, pills, dragees, capsules, liquids, gels, syrups, slurries, suspensions and the like, for oral ingestion by a subject. In certain embodiments, pharmaceutical compositions for oral use are obtained by mixing oligonucleotide and one or more solid excipient. Suitable excipients include, but are not limited to, fillers, such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). In certain embodiments, such a mixture is optionally ground and auxiliaries are optionally added. In certain embodiments, pharmaceutical compositions are formed to obtain tablets or dragee cores. In certain embodiments, disintegrating agents (e.g., cross-linked polyvinyl pyrrolidone, agar, or alginic acid or a salt thereof, such as sodium alginate) are added.

In some embodiments, dragee cores are provided with coatings. In certain such embodiments, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to tablets or dragee coatings.

In other embodiments, pharmaceutical compositions for oral administration are push-fit capsules made of gelatin. Certain of such push-fit capsules comprise one or more pharmaceutical agents of the present invention in admixture with one or more filler such as lactose, binders such as starches, and/or lubricants such as talc or magnesium stearate and, optionally, stabilizers. In certain embodiments, pharmaceutical compositions for oral administration are soft, sealed capsules made of gelatin and a plasticizer, such as glycerol or sorbitol. In certain soft capsules, one or more pharmaceutical agents of the present invention are be dissolved or suspended in suitable liquids, such as fatty oils, liquid paraffin, or liquid polyethylene glycols. In addition, stabilizers may be added.

In certain embodiments, pharmaceutical compositions are prepared for buccal administration. Certain of such pharmaceutical compositions are tablets or lozenges formulated in conventional manner.

In preferred embodiments, a pharmaceutical composition is prepared for administration by injection (e.g., intravenous, subcutaneous, intramuscular, etc.). In certain of such embodiments, a pharmaceutical composition comprises a carrier and is formulated in aqueous solution, such as water or physiologically compatible buffers such as Hanks's solution, Ringer's solution, or physiological saline buffer. In certain embodiments, other ingredients are included (e.g., ingredients that aid in solubility or serve as preservatives). In certain embodiments, injectable suspensions are prepared using appropriate liquid carriers, suspending agents and the like. Certain pharmaceutical compositions for injection are presented in unit dosage form, e.g., in ampoules or in multi-dose containers. Certain pharmaceutical compositions for injection are suspensions, solutions or emulsions in oily or aqueous vehicles, and may contain formulatory agents such as suspending, stabilizing and/or dispersing agents. Certain solvents suitable for use in pharmaceutical compositions for injection include, but are not limited to, lipophilic solvents and fatty oils, such as sesame oil, synthetic fatty acid esters, such as ethyl oleate or triglycerides, and liposomes. Aqueous injection suspensions may contain substances that increase the viscosity of the suspension, such as sodium carboxymethyl cellulose, sorbitol, or dextran. Optionally, such suspensions may also contain suitable stabilizers or agents that increase the solubility of the pharmaceutical agents to allow for the preparation of highly concentrated solutions.

In certain embodiments, a pharmaceutical composition is prepared for transmucosal administration. In certain of such embodiments penetrants appropriate to the barrier to be permeated are used in the formulation. Such penetrants are generally known in the art.

In certain embodiments, a pharmaceutical composition is prepared for administration by inhalation. Certain of such pharmaceutical compositions for inhalation are prepared in the form of an aerosol spray in a pressurized pack or a nebulizer. Certain of such pharmaceutical compositions comprise a propellant, e.g., dichlorodifluoromethane, trichlorofluoromethane, dichlorotetrafluoroethane, carbon dioxide or other suitable gas. In certain embodiments using a pressurized aerosol, the dosage unit may be determined with a valve that delivers a metered amount. In certain embodiments, capsules and cartridges for use in an inhaler or insufflator may be formulated. Certain of such formulations comprise a powder mixture of a pharmaceutical agent of the invention and a suitable powder base such as lactose or starch.

In some embodiments, a pharmaceutical composition is prepared for rectal administration, such as a suppositories or retention enema. Certain of such pharmaceutical compositions comprise known ingredients, such as cocoa butter and/or other glycerides.

In other embodiments, a pharmaceutical composition is prepared for topical administration. Certain of such pharmaceutical compositions comprise bland moisturizing bases, such as ointments or creams. Exemplary suitable ointment bases include, but are not limited to, petrolatum, petrolatum plus volatile silicones, and lanolin and water in oil emulsions. Exemplary suitable cream bases include, but are not limited to, cold cream and hydrophilic ointment.

in certain embodiments, a pharmaceutical composition of the present invention comprises a modified oligonucleotide in a therapeutically effective amount. Usually the therapeutically effective amount is sufficient to prevent, alleviate or ameliorate symptoms of a disease or to prolong the survival of the subject being treated. Determination of a therapeutically effective amount is well within the capability of those skilled in the art.

In a particularly preferred embodiment the pharmaceutical composition of the present invention is applied to a stent which is to be used in the treatment of endovascular problems and/or diseases. The stents usually comprise a supporting structure consisting of inert metals and/or plastic material which does not cause complications when in contact with the cells of the human body. Such supportive material which is known to the person skilled in the art is frequently covered with a coating. Such coating must also be compatible with the immune system when in contact with the cells and tissues of the human body. The coating serves as a matrix comprising the pharmaceutical composition of the present invention. The matrix may either be permanently maintained or the matrix may also be degraded over the time. Depending on the material selected, the degradation of the matrix can vary. Such a type of matrix may be formed by using biodegradable polymers like polylactide, polylactide/polyglycolide and the like.

In certain embodiments, one or more modified oligonucleotides of the present invention is formulated as a prodrug. In certain embodiments, upon *in vivo* administration, a prodrug is chemically converted to the biologically, pharmaceutically or therapeutically more active form of a modified oligonucleotide. In certain embodiments, prodrugs are useful because they are easier to administer than the corresponding active form. For example, in certain instances, a prodrug may be more bioavailable (e.g., through oral administration) than is the corresponding active form. In certain instances, a prodrug may have improved solubility compared to the corresponding active form. In certain embodiments, prodrugs are less water soluble than the corresponding active form. In certain instances, such prodrugs possess superior transmittal across cell membranes, where water solubility is detrimental to mobility. In certain embodiments, a prodrug is an ester. In certain such embodiments, the ester is metabolically hydrolyzed to carboxylic acid upon administration. In certain instances the carboxylic acid containing compound is the corresponding active form. In certain embodiments, a prodrug comprises a short peptide (polyamifloacid) bound to an acid group. In certain of such embodiments, the peptide is cleaved upon administration to form the corresponding active form.

In certain embodiments, a prodrug is produced by modifying a pharmaceutically active compound such that the active compound will be regenerated upon *in vivo* administration. The prodrug can be designed to alter the metabolic stability or the transport characteristics of a drug, to mask side effects or toxicity, to improve the flavor of a drug or to alter other characteristics or properties of a drug. By virtue of knowledge of pharmacodynamic processes and drug metabolism *in vivo,* those of skill in this art, once a pharmaceutically active compound is known, can design prodrugs of the compound (see, e.g., Nogrady (1985) Medicinal Chemistry A Biochemical Approach, Oxford University Press, New York, pages 388-392).

The present invention is further illustrated by the following examples.

### Example 1:

### Microarray results and miR-100 expression pattern

miRNA-microarray analysis was performed with total RNA-isolations of hindlimb tissue from the distal thigh at baseline and five timepoints following femoral artery occlusion.

Total RNA was isolated from the distal adductor muscles by phenol-chloroform isolation (TRlzol, Invitrogen) at six timepoints following femoral artery ligation from five mice per timepoint. RNA was pooled in equal amounts and microarray assay for all identified murine miRNAs at this time (miRBase 9.0) was performed using a service provider (LC Sciences, Houston, Texas).

Since the major part of the analyzed RNA-samples is derived not from the growing vasculature but the surrounding tissue, a combined analysis of all five timepoints following femoral artery ligation was chosen for target selection. Instead of choosing miRNAs with a strong change in expression at one timepoint for further analysis, for the first screening approach it was focused specifically on miRNAs with a persistent trend in differential expression over several time points. Overall, 19 upregulated miRNAs and 16 downregulated miRNAs with a statistically significant (p<0.01) change in expression and a clear trend over several time points were identified.

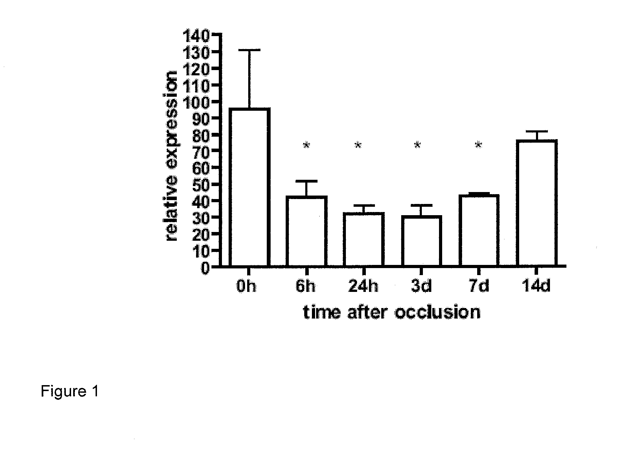

Among the miRNAs with a continuously decreasing expression until day 3, followed by a recovery of expression up to near-baseline levels at day 14, was the microRNA miR-100. RT-PCR using miRNA stem loop primers for miR-100 on the individual RNA-samples were in good agreement with the microarray data (Figure 1).

Using in situ hybridization in combination with immunofluorescent staining for vascular marker, miR-100 expression was detected in both endothelial as well as in vascular smooth muscle cells, as well as in a variety of perivascular cell types.

Due to the qualitative approach of the in-situ hybridization technique, changes in expression levels following femoral artery occlusion could not be trustingly quantified. To investigate if the observed change in expression was indeed related to vascular structures, collateral arteries including the adventitial tissue from the murine hindlimb at 3 days following femoral artery occlusion for RT-PCR analysis were isolated. miR-100 was expressed in both growing collateral arteries (proliferation verified by Ki67-expression) as well as in the surrounding musculature without visible arteries. However, miR-100-expression was significantly decreased in growing collateral arteries compared to the quiescent blood vessels from the sham-operated side.

miR-100 is not expressed in an organ-specific manner, but is detectable by stem-loop RT-PCR in a wide selection of tissues.

### Example 2:

### Modulation of miR-100 expression in cultured endothelial and vascular smooth muscle cells

To verify the expression of miR-100 in pure cell populations of the vascular wall, stem-loop RT-PCR was used for total RNA-isolates from cultured human endothelial cells (HUVECs) and human aortic vascular smooth muscle cells.

MicoRNA expression was validated by quantitative stem-loop PCR technology (TaqMan MicroRNA Assays, Applied Biosystems, Foster City, California). The use of target-specific reverse transcription primers and TaqMan hybridization probes allows the specific detection of mature microRNAs. MicroRNA expression was normalized to the expression of the small RNAs rnu19 and rnu48.

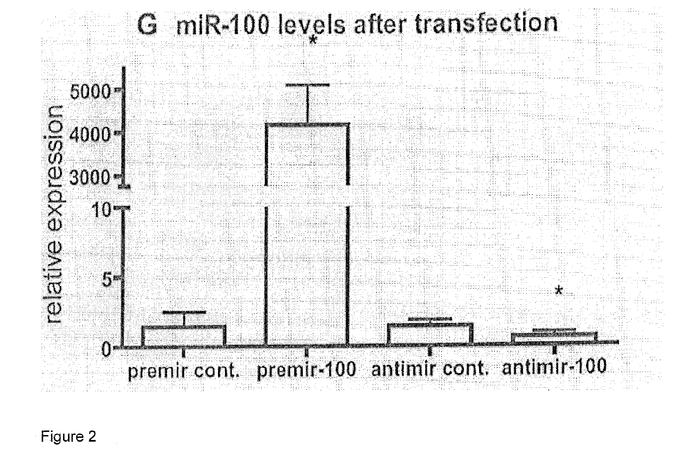

miR-100 was strongly expressed in both cell types. To investigate a potential role of miR-100 for angiogenesis, miR-100 was overexpressed and silenced in these cell types by transfecting specific miR-100 precursors (pre-miRs) or antisense-molecules (anti-miRs), respectively. An optimized transfection protocol using fluorescent control pre- and anti-miRNAs showed a transfection efficiency of more than 90%. Quantitative RT-PCR revealed a strong increase in detectable miR-100 at 24h following pre-miR-100 transfection, whereas transfection with anti-miR-100 resulted in significantly decreased miR-100 levels (Figure 2). Pre- and anti-miRs with an irrelevant nucleotide sequence served as negative controls. There was no significant difference in cellular viability following transfection with the different compounds.

The overexpression of miR-100 had a significant inhibitory effect on both endothelial network formation in the planar matrigel assay and endothelial sprouting in the three-dimensional spheroid culture assay. In both assays, the reduction of miR-100 levels by transfection of anti-miRs had a stimulatory effect, resulting in an increased endothelial network formation and a longer total sprout length. No significant difference in migratory speed was found in the planar wound healing migration assay.

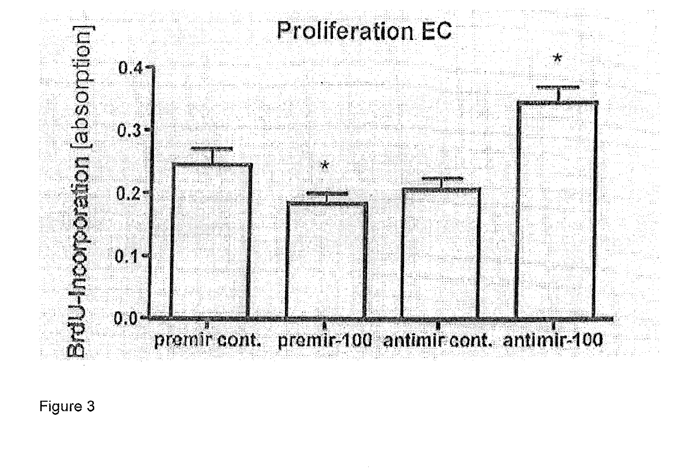

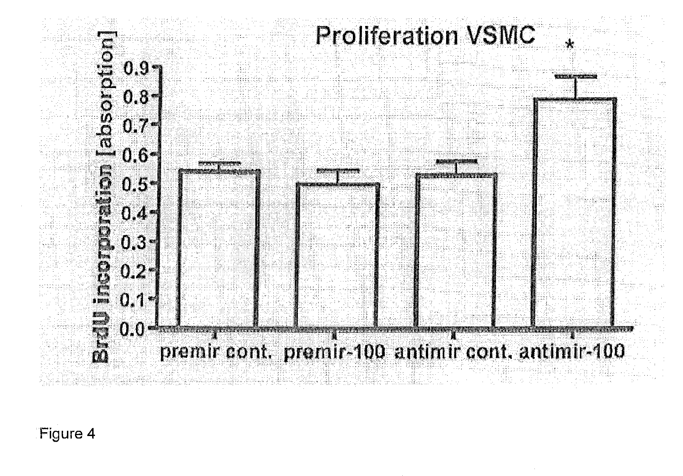

To assess whether the observed differences were secondary to effects on cellular viability, endothelial cell apoptosis and proliferation following miR-100 modulation was checked. Using flow cytometric quantification of Annexin V-staining a slight increase in endothelial cell apoptosis following miR-100 overexpression was detected compared to an irrelevant control sequence. Silencing of miR-100 by anti-miR transfection however had no significant effect compared to the proper control, therefore making an anti-apoptotic effect of miR-100-inhibition an unlikely explanation for the observed differences in in-vitro angiogenesis. DNA-synthesis rate was further analyzed by measuring BrdU-incorporation, which correlates well with cellular proliferation. A significant attenuation of endothelial cell proliferation by pre-miR-100 transfection was found, whereas inhibition of miR-100 by anti-miRs had a consistent stimulatory effect (Figure 3). The inhibitory effect of pre-miR-100 transfection was more pronounced in endothelial cells than in smooth muscle cells, possibly due to the lower baseline-levels of miR-100 in these cells (Figure 4).

### Example 3:

### miR-100 modulates proliferation by targeting m TOR

Using a combination of bioinformatic prediction algorithms and microarray based gene expression analysis of endothelial cells following miR-100 overexpression, it was searched for potential target genes of miR-100 that could be responsible for the observed effects. The mammalian target of rapamycin (mTOR, coded by the FRAP1-gene) was identified, which was downregulated following premiR-100 transfection and which was predicted to be direct target of this microRNA by three different algorithms (PicTar, Targetscan, Miranda).

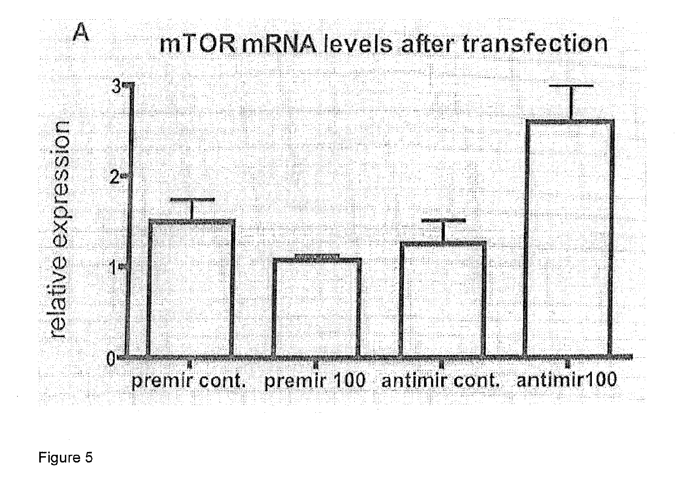

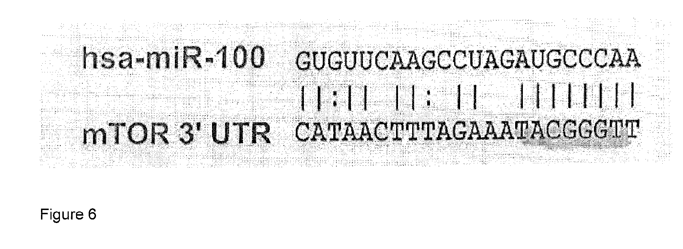

It was found that overexpression of miR-100 significantly repressed mTOR-expression on both mRNA as well as protein level in endothelial cells, whereas miR-100 inhibtion resulted in a significant upregulation (Figure 5). The binding site of miR-100 in the 3'-UTR of mTOR is shown in (Figure 6.) In Figure 6 the sequence designated hsa-miR-100 corresponds to SEQ ID N0:1 and the sequence having the designation mTOR 3'UTR corresponds to SEQ ID N0:7.

### Example 4:

### Interaction between mTOR and miRNA-100

To investigate if the repression of mTOR was responsible for the observed attenuation of cellular proliferation by miR-100, a rescue-experiment was performed by transfecting mTOR-coding plasmids in combination with pre-miR-100.

For transfection with pre-miR microRNA precursor molecules or anti-miRmiRNA inhibitors (all Ambion), cells were cultured to 70% confluence and transfected with 8nM pre-miR-100 or anti-miR-100 oligonucleotides using Lipofectamin RNAiMax (invitrogen) according to the manufacturers instructions. For transfection of HEK293 cells with mTOR plasmids, 2µg DNA per 3x10E5 cells was transfected with Fugene 6 transfection reagent (Roche). For double transfection of plasmids with miRNA-precursors and inhibitors, pre- and anti-miRs were added to the plasmid/Fugene transfection mix in the same final concentrations as stated above. mTOR (Acc. NM004958) full length (SKU SC124066) and ORF-constructs (SKU RC220457) were obtained from the Origene collection (Origene, Rockville, MD).

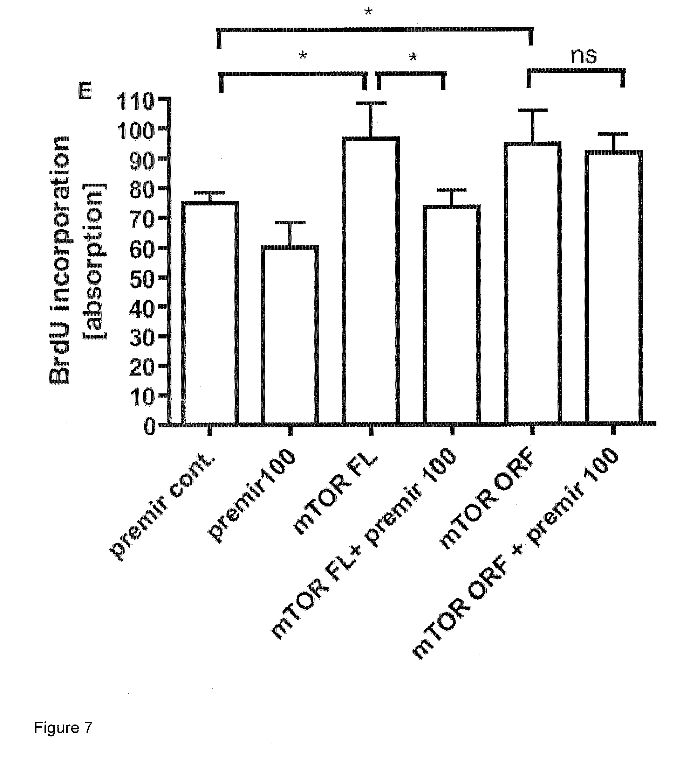

Due to the large plasmid size of mTOR, it was not possible to achieve a sufficient transfection rate in endothelial cells without procedure-related toxic effects and therefore the easily transfected HEK-cells were chosen as a culture model, where the miR-100 effects on proliferation seen in endothelial cells could be reproduced (Figure 7). Whereas overexpression of full-length mTOR including the 3'UTR was able to partially rescue the anti-proliferative effect of premiR-1 00 transfection, a complete rescue could be achieved by expressing an ORF-clone lacking the miR-100 binding site. Matching the decreased expression of miR-100 in the ischemic hindlimb, immunofluorescent staining showed a stronger expression of mTOR, predominantly in CD31 positive endothelial cells of the ischemic hindlimb.

### Example 5:

### Inhibition of miR-100 in vivo by antagomir-treatment stimulates perfusion restoration in mice.

The murine hindlimb ischemia model conforms to the Guide for the Care and Use of Laboratory Animals published by the U.S. National Institutes of Health and was performed after securing appropriate institutional approval. Unilateral femoral artery occlusion was performed in C57/B16J mice (Charles River Lab., Sulzfeld, Germany) by double ligation of the superficial femoral artery proximal to the deep femoral artery and the distal femoral artery just above the knee joint. A sham operation without arterial occlusion was performed on the contralateral leg. For molecular biology, histology and in situ hybridization, tissue was carefully dissected and snap-frozen in liquid nitrogen until further analysis. For dissection of isolated collateral arteries, the hindlimb vasculature was performed with liquid latex, to allow the identification and isolation of individual arterial vessels.

The mouse model is well established and provides a reliable prediction for the situation in humans (Seiler et al. Circulation 2001, 104, 2012-2017, or Limbourg et al, Nature Protocols 2009, Vol. 4, No. 12, 1737-1748).

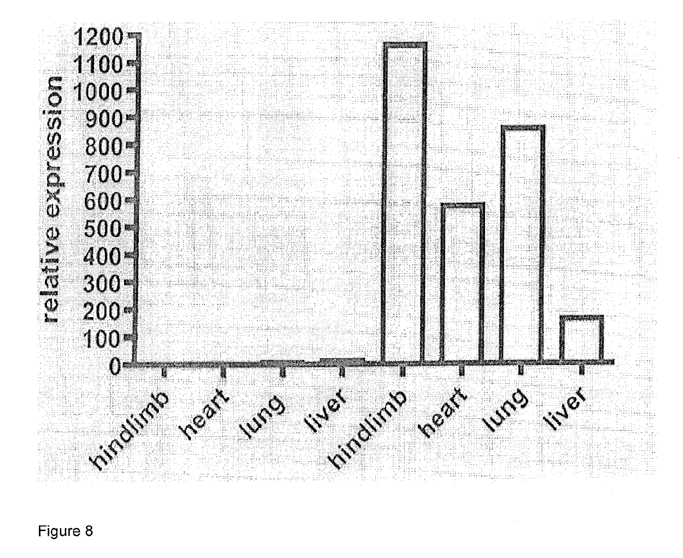

To assess whether inhibition of miR-100 can be be used to stimulate revascularization in vivo, mice were treated with a specific antagomir against miR-100 after induction of unilateral hindlimb ischemia. Three doses of antagomir resulted in a strong and sustained suppression of miR-100 expression in a wide selection of tissues (Figure 8) and fluorescence-labeled antagomir could be found in the endothelial layer of blood vessels. The most prominent accumulation was found in liver and lung sections.

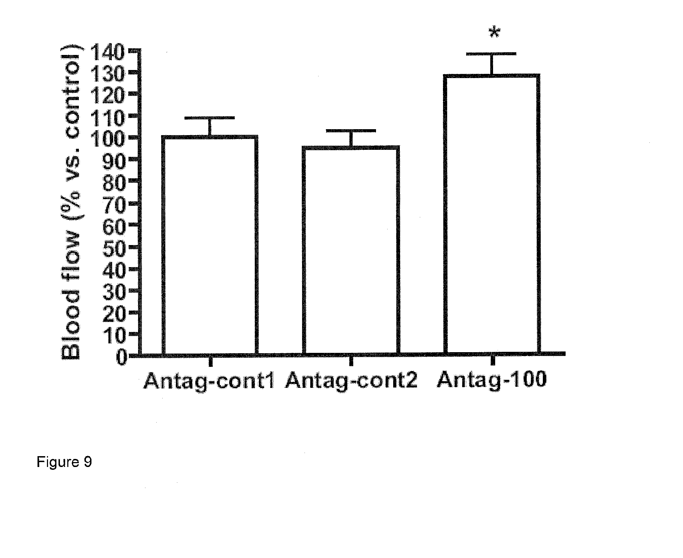

Antagomir treatment resulted in a significant stimulation of perfusion restoration, with higher flow ratio at day 7 following induction of ischemia, compared to treatment with two control-antagomirs, one constructed to represent a scrambled version of the anti-miR-100 antagomir and one that was previously shown to be without effect on perfusion restoration in this model (Figure 9). This functional difference was also detectable on a morphological level, where miR-100 inhibition resulted in an increase in capillary density in the distal hindlimb tissue. This comparative example demonstrates clearly the specific effectiveness of the antagomirs of the present invention.

### Example 6:

### Inhibition of mTOR impairs angiogenesis in mice

In addition, we treated two separate groups of mice with daily injections of the mTOR-inhibitor rapamycin or solvent to investigate the timecourse of hindlimb perfusion restoration under conditions of endogenous miR-100 downregulation, but impaired signalling of its target gene mTOR. Rapamycin treatment resulted in a significant attenuation of blood flow restoration at day 7 after femoral artery ligation and a decreased capillary density in the ischemic tissue.

### Example 7:

### miR-100 in endothelial cells is downregulated in response to TNF-alpha in a NFkappaB-dependent manner.

To search for possible inducers of miR-100 downregulation in endothelial cells, several pro-angiogenic or pro-arteriogenic cytokines were screened for effects on miR-100 expression. Whereas VEGF, TGF-beta1 and FGF2 had no sustained and reproducible effect in the concentrations tested, TNF-alpha resulted in a significant downregulation of miR-100 after prolonged exposure in a time dependent manner. We did not find a statistically significant dose dependency in the concentrations tested, but a trend for a more pronounced decrease of miR-100 expression with higher doses of TNF-alpha. Blockade of the NF-kappaB pathway by pre-incubation with the IkB kinase- inhibitor PS1145 abrogated the TNF-alpha effect. Expression of TNF-alpha was significantly upregulated in the hindlimb tissue following femoral artery ligation in mice, corresponding to the detected downregulation of miR-100 in vivo. lmmunhistochemistry showed a strong accumulation of F4/80 positive cells, which are presumed to be macrophages, in the perivascular space in the occluded hindlimb, which could serve as the endogenous source of TNF-alpha in the ischemic tissue.

Organization Applicant
---------------------
      Street : Hugstetterstr. 49
      City : Freiburg
      State :
      Country : Germany
      PostalCode : 79106
      PhoneNumber :
      FaxNumber :
      EmailAddress :
<110> OrganizationName : Universitätsklinikum Freiburg

Application Project
-------------------
<120> Title : Pharmaceutical composition comprising miRNA-100 and its use in
       the modulation of blood vessel growth
<130> AppFileReference : ZEE20090121
<140> CurrentAppNumber :
<141> CurrentFilingDate : ____-__-__

Sequence
--------
<213> OrganismName : Homo sapiens
<400> PreSequenceString :
guguucaagc cuagaugccc aa                                                22
<212> Type : RNA
<211> Length : 22
      SequenceName : 1
      SequenceDescription :miRNA100

Sequence
--------
<213> OrganismName : artificial sequence
<400> PreSequenceString :
gtgttcaagc ctagatgccc aa                                                22
<212> Type : DNA
<211> Length : 22
      SequenceName : 2
      SequenceDescription :DNA sequence of miRNA100

Sequence
--------
<213> OrganismName : artificial sequence
<400> PreSequenceString :
cacaaguucg gaucuacggg uu                                                22
<212> Type : RNA
<211> Length : 22
      SequenceName : 3
      SequenceDescription : antagomir of miRNA100

Sequence
--------
<213> OrganismName : artificial sequence
<400> PreSequenceString :
aaggcaagcu gacccugaag uu                                                22
<212> Type : RNA
<211> Length : 22
      SequenceName : 4
      SequenceDescription : control antagomir

Sequence
--------
<213> OrganismName : artificial sequence
<400> PreSequenceString :
caccaguuag gcucuacgga uu                                                22
<212> Type : RNA
<211> Length : 22
      SequenceName : 5
      SequenceDescription : control antagomir

Sequence
--------
<213> OrganismName : artificial sequence
<400> PreSequenceString :
uacgggu 7
<212> Type : RNA
<211> Length : 7
SequenceName : 6
SequenceDescription : conserved region

Sequence
--------
<213> OrganismName : Homo sapiens
<400> PreSequenceString :
cataacttta gaaatacggg tt 22
<212> Type : DNA
<211> Length : 22
SequenceName : 7
SequenceDescription : mTOR 3´UTR

## Claims

1. Pharmaceutical composition comprising a miRNA-100 molecule or an antagomir thereof or a variant thereof.

2. Pharmaceutical composition according to claim 1 for the positive or negative modulation of blood vessel growth.

3. Pharmaceutical composition according to claim 1 for the modulation of proliferation, tube-formation and sprouting activity of endothelial cells.

4. Pharmaceutical composition according to any of claims 1 to 3, **characterized in that** the miRNA-100 molecule is an oligonucleotide comprising at least 20 nucleotides of any of the sequences SEQ ID No. 1, SEQ ID No. 2, SEQ ID No. 3, or a variant thereof.

5. Pharmaceutical composition according to any of claims 1 to 3, **characterized in that** the miRNA-100 molecule or antagomir or variant thereof is an oligonucleotide comprising at least 20 nucleotides complementary to a sequence of SEQ ID No. 1, SEQ ID No. 2 or SEQ ID No. 3, or a variant thereof.

6. Pharmaceutical composition according to claim 5, **characterized in that** the miRNA-100 antagomir or variant thereof is an oligonucleotide having at least 85% homology to SEQ ID NO:3.

7. Pharmaceutical composition according to claim 5 **characterized in that** the miRNA-100 molecule or variant thereof is an oligonucleotide having at least 85% homology to SEQ ID N0:1 or SEQ ID NO:2.

8. Pharmaceutical composition according to any of claims 4 to 7 **characterized in that** the oligonucleotide is an RNA molecule which may optionally be chemically modified.

9. Pharmaceutical composition according to claim 4 to 7, **characterized in that** the miRNA-100 molecule or antagomir or variant thereof is modified.

10. Pharmaceutical composition according to any of claims 4 to 6, **characterized in that** the miRNA-100 sequence or the complementary sequence thereof is contained in vector replicable in the patient.

11. Pharmaceutical composition according to any one of claims 1 to 10 **characterized in that** it is applied in a stent or the coating of a stent to be used for the treatment of endovascular conditions.

12. Use of miRNA-100 molecule, an antagomir thereto or a variant thereof for the manufacture of a medicament for the modulation of blood vessel growth.

13. Use according to claim 12, wherein the proliferation, tube-forming and sprouting activity of endothelial cells is enhanced.

14. Use according to claims 12 or 13 wherein the miRNA-100 molecule or antagomir thereof or variant thereof comprises at least 20 nucleotides of any of the sequences SEQ ID N0:1, SEQ ID NO:2 and SEQ ID NO:3 or a nucleotide sequence which has a homology of at least 85% to SEQ ID N0:1, SEQ ID NO:2 and/or SEQ ID NO:3.

15. Use according to claim 14 wherein the oligonucleotide is an RNA molecule which is optionally modified.
